(19) Europäisches Patentamt
European Patent Office
Office européen des brevets

(11) Veröffentlichungsnummer: **0 101 800**
**A1**

(12) # EUROPÄISCHE PATENTANMELDUNG

(21) Anmeldenummer: 83105502.5

(22) Anmeldetag: 03.06.83

(51) Int. Cl.³: C 07 D 233/80
C 07 D 235/02, A 01 N 43/50
A 01 N 43/52

(30) Priorität: 16.06.82 DE 3222523

(43) Veröffentlichungstag der Anmeldung: 07.03.84 Patentblatt 84/10

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Kühle, Engelbert, Dr.
von Bodelschwingh-Strasse 42
D-5060 Bergisch-Gladbach 2(DE)

(72) Erfinder: Brandes, Wilhelm, Dr.
Eichendorffstrasse 3
D-5653 Leichlingen 1(DE)

(72) Erfinder: Rosslenbroich, Hans-Jürgen, Dr.
Am Hagelkreuz 2
D-4018 Lengenfeld(DE)

(72) Erfinder: Klauke, Erich, Dr.
Eichendorffweg 8
D-5068 Odenthal(DE)

(54) **N-Sulfenylierte Hydantoine.**

(57) Die vorliegende Erfindung betrifft neue N-sulfenylierte Hydantoine der Formel (I)

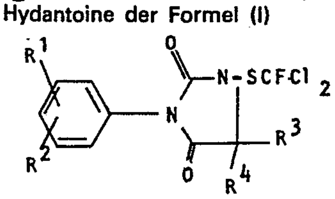

(I)

in welcher

$R^1$ für Trihalogenmethoxy oder Trihalogenmethylmercapto steht,

$R^2$ für Wasserstoff, Nitro oder Halogen steht und

$R^3$ und $R^4$ gleich oder verschieden sind und für Wasserstoff oder Alkyl stehen oder gemeinsam mit dem Ringkohlenstoff an dem sie stehen einen fünf- oder sechsgliedrigen Cycloalkylrest bilden.

Man kann die neuen Verbindungen durch Umsetzung entsprechend substituierter Hydantoine mit Dichlorfluormethansulfenylhalogenid erhalten. Die neuen N-sulfenylierten Hydantoine der Formel (I) können als Schädlingsbekämpfungsmittel eingesetzt werden.

EP 0 101 800 A1

BAYER AKTIENGESELLSCHAFT 5090 Leverkusen, Bayerwerk

Zentralbereich
Patente, Marken und Lizenzen Bas/ABc

Ia

## N-Sulfenylierte Hydantoine

Die Erfindung betrifft neue N-sulfenylierte Hydantoine, ein Verfahren zu ihrer Herstellung sowie ihre Verwendung als Schädlingsbekämpfungsmittel.

Es ist bereits bekannt, daß bestimmte N-Dichlorfluormethansulfenyl-hydantoine als Fungizide in der Landwirtschaft verwendet werden können (vgl. US-PS 3 960 883). Weiterhin sind seit langem Schwermetallsalze der Ethylen-1,2-bis-dithiocarbaminsäure, insbesondere das Zink-ethylen-1,2-bis-dithiocarbamat, in Landwirtschaft und Gartenbau zur Bekämpfung von pflanzenpathogenen Pilzen in Gebrauch (vgl. R. Wegler, "Chemie der Pflanzenschutz- und Schädlingsbekämpfungsmittel", Band 2, Seite 65, Springer-Verlag Berlin/Heidelberg/New York (1970)).

Es wurden neue N-Dichlorfluormethansulfenylhydantoine der Formel

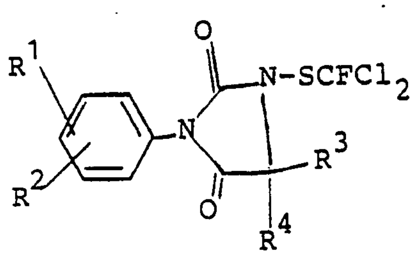

(I)

gefunden, in welcher

$R^1$ für Trihalogenmethoxy oder Trihalogenmethylmercapto steht,

$R^2$ für Wasserstoff, Nitro oder Halogen steht und

$R^3$ und $R^4$ gleich oder verschieden sind und für Wasserstoff oder Alkyl stehen oder gemeinsam mit dem Ringkohlenstoff einen fünf- oder sechsgliedrigen Cycloalkylrest bilden.

Man erhält die neuen N-sulfenylierten Hydantoine der Formel (I)

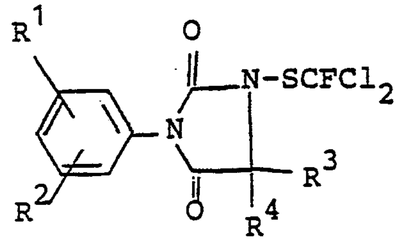

(I)

in welcher

$R^1$ für Trihalogenmethoxy oder Trihalogenmethylmercapto steht,

$R^2$ für Wasserstoff, Nitro oder Halogen steht und

$R^3$ und $R^4$ gleich oder verschieden sind und für Wasserstoff oder Alkyl stehen oder gemeinsam mit dem Ringkohlenstoff an dem sie stehen einen fünf- oder sechsgliedrigen Cycloalkylrest bilden, wenn man ein Hydantoin der Formel

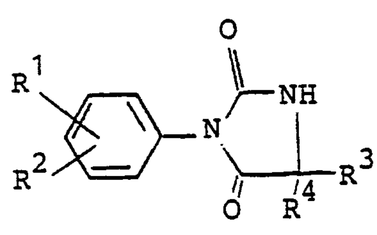

(II)

in welcher
$R^1$, $R^2$, $R^3$ und $R^4$ die oben angegebene Bedeutung haben, mit einem Dichlorfluormethansulfenylhalogenid der Formel

$$X\text{-}SCCl_2F \qquad (III)$$

in welcher
X für Halogen steht,

gegebenenfalls in Gegenwart eines säurebindenden Mittels und eines Verdünnungsmittels umsetzt.

Die neuen N-sulfenylierten Hydantoine der Formel (I) besitzen fungizide Eigenschaften. Überraschenderweise zeigen sie eine Überlegenheit in ihrer Wirkung gegenüber den bekannten Verbindungen. Sie stellen somit eine Bereicherung der Technik dar.

Die erfindungsgemäßen N-sulfenylierten Hydantoine sind durch die Formel (I) definiert. In dieser Formel stehen vorzugsweise

$R^1$ für Trichlormethoxy, Trifluormethoxy, Trichlormethylmercapto, Trifluormethylmercapto, Difluorchlormethoxy, Fluordichlormethoxy, Difluorchlormethylmercapto oder Fluordichlormethylmercapto,

$R^2$ für Wasserstoff, Nitro oder Chlor,

$R^3$ und $R^4$ gleich oder verschieden sind und für Wasserstoff, Niederalkyl mit 1 bis 4 Kohlenstoffatomen oder gemeinsam mit dem Ringkohlenstoffatom an dem sie stehen für Cyclopentyl oder Cyclohexyl.

Besonders bevorzugt sind die Verbindungen der Formel (I), in denen

$R^1$ für Trichlormethoxy, Trifluormethoxy, Trichlormethylmercapto, Trifluormethylmercapto, Difluorchlormethoxy, Fluordichlormethoxy, Difluorchlormethylmercapto oder Fluordichlormethylmercapto,

$R^2$ für Wasserstoff oder Chlor,

$R^3$ und $R^4$ gleich sind und für Wasserstoff oder Niederalkyl mit 1 bis 3 Kohlenstoffatomen, wie Methyl, Ethyl, iso-Propyl und n-Propyl, stehen oder gemeinsam mit dem Ringkohlenstoff einen Cyclopentyl oder Cyclohexylrest bilden.

Verwendet man zur Herstellung der erfindungsgemäßen Verbindungen beispielsweise 3-(4-Trifluormethoxyphenyl)-hydantoin und Dichlorfluormethansulfenylchlorid als Ausgangsmaterialien, so kann der Reaktionsverlauf durch das nachfolgende Reaktionsschema wiedergegeben werden:

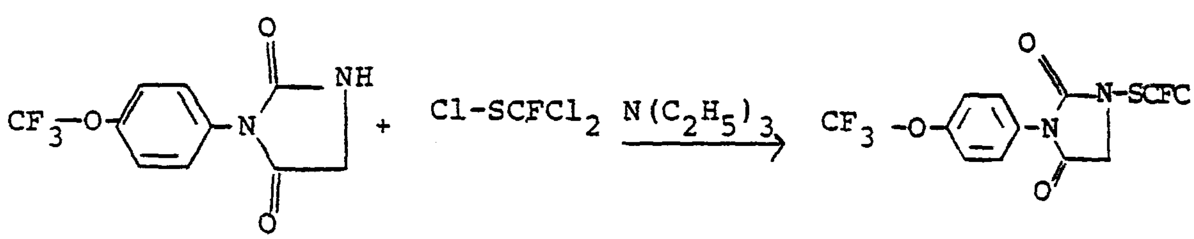

Die bei der Durchführung des erfindungsgemäßen Verfahrens als Ausgangsstoffe zu verwendenden Dichlorfluormethansulfenylhalogenide sind durch die Formel (III) definiert. In dieser Formel (III) steht X für Halogen, wie Chlor oder Brom, vorzugsweise für Chlor.

Die Dichlorfluormethansulfenylhalogenide sind bekannt und nach bekannten Verfahren herstellbar /vgl. z.B. Angew. Chemie 76, Seite 807 (1964)/.

Die außerdem als Ausgangsmaterialien zu verwendenden Hydantoine sind durch die Formel (II) definiert. In dieser Formel (II) stehen $R^1$ bis $R^4$ für die Reste, die bereits im Zusammenhang mit der Beschreibung der erfindungsgemäßen Stoffe der Formel (I) für diese Substituenten genannt wurden.

Diese Hydantoine der Formel (II) sind größtenteils bekannt und nach bekannten Verfahren herstellbar, so z.B. durch Umsetzung der entsprechenden Phenylisocyanate mit Aminoessigsäure und nachfolgende Cyclisierung unter Wasserabspaltung

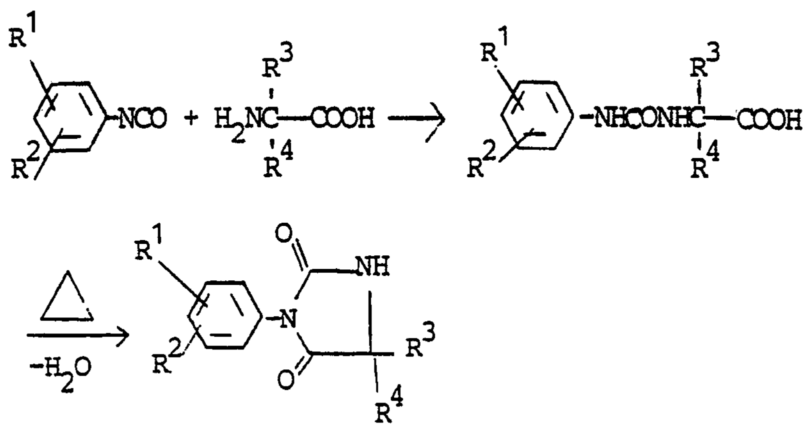

oder durch Addition von entsprechenden Aminoacetonitrilen an Isocyanate und nachfolgende Cyclisierung und Verseifung des Iminorestes, z.B.

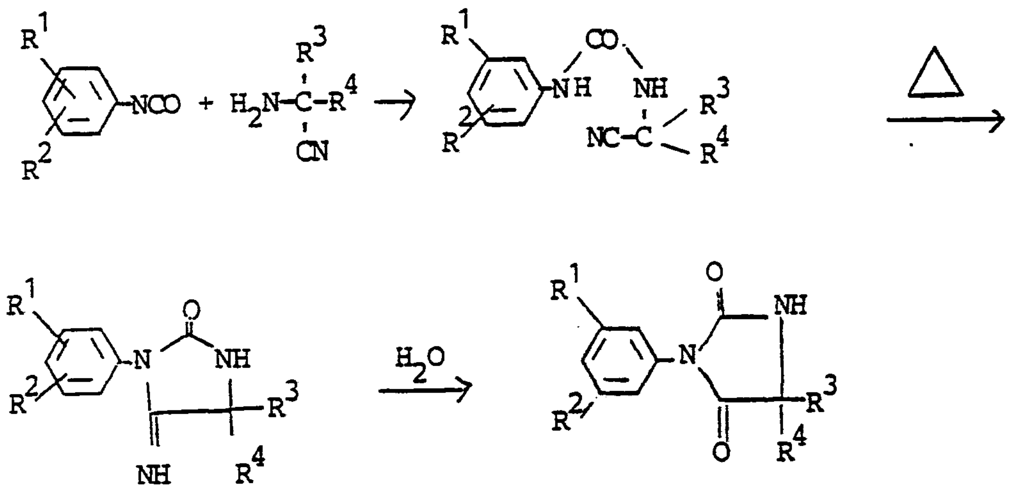


(vgl. Chem. Abstracts 55, 27277 h (1961)).

Bei der Durchführung des Verfahrens kommen als Verdünnungsmittel alle inerten organischen Lösungsmittel in Frage. Hierzu gehören vorzugsweise Kohlenwasserstoffe wie Toluol, Chlorkohlenwasserstoffe wie Chlorbenzol, Ether wie Dioxan. Man kann die Umsetzung aber auch in Wasser durchführen.

Als säurebindende Mittel können tert. Amine sowie Alkalihydroxide oder Alkalicarbonate wie Natriumhydroxid oder Natriumcarbonat verwendet werden.

Die Reaktionstemperaturen können in einem größeren Bereich variiert werden. Im allgemeinen arbeitet man zwischen etwa 0°C und 100°C, vorzugsweise bei 20 bis 50°C.

Bei der Durchführung der Umsetzung setzt man auf 1 Mol des entsprechenden Hydantoins 1 Mol Trihalogenmethansulfenylhalogenid ein. Ein geringer Überschuß an Sulfenylhalogenid von bis zu 3 % schadet nicht. Die Isolierung der Endprodukte der Formel (I) erfolgt in allgemein üblicher Art und Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet.

So werden z.B. fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä.,

sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage:

z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trocknenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

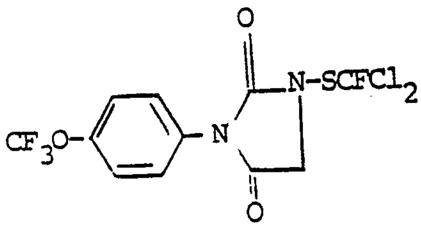

18,2 g (0,07 Mol) 3-(4-Trifluormethoxyphenyl)-hydantoin werden unter Zugabe von 12,5 g (0,074 Mol) Dichlorfluormethansulfenylchlorid in 100 ml Dioxan gelöst. Dazu tropft man 8 g (0,079 Mol) Triethylamin. Hierbei steigt die Temperatur bis 53°C an. Man rührt etwa 15 Min. und fällt das Produkt durch Zugabe von Wasser aus. Man erhält 11 g (40 % der Theorie) an 1-Dichlorfluormethylmercapto-3-(4-trifluormethoxy-phenyl)-hydantoin mit dem Schmelzpunkt 153-156°C.

In ähnlicher Weise erhält man folgenden Verbindungen der Formel I

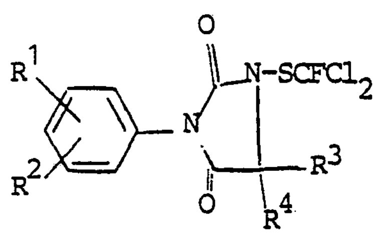

(I)

| Bsp.-Nr. | $R^1$ | $R^2$ | $R^3$ | $R^4$ | Schmelzpunkt (°C) |
|---|---|---|---|---|---|
| 2 | $4-F_3CO-$ | H | $CH_3$ | $CH_3$ | 85 |
| 3 | $4-ClF_2C-O-$ | H | H | H | 152 |
| 4 | $4-ClF_2-C-O-$ | H | $CH_3$ | $CH_3$ | 88 |

| Bsp. Nr. | $R^1$ | $R^2$ | $R^3$ | $R^4$ | Schmelzpunkt (°C) |
|---|---|---|---|---|---|
| 5 | $4-F_3C-S-$ | H | H | H | 150 |
| 6 | $4-F_3C-S-$ | H | $CH_3$ | $CH_3$ | 94 |
| 7 | $4-F_3C-O-$ | 3-Cl | H | H | 98 |
| 8 | $4-F_3C-O-$ | 3-Cl | $CH_3$ | $CH_3$ | 82 |
| 9 | $4-ClF_2C-O-$ | 3-Cl | H | H | 85 |
| 10 | $4-ClF_2C-S-$ | 3-Cl | H | H | 87 |

Herstellungsbeispiele der Ausgangsverbindungen

Beispiel a:

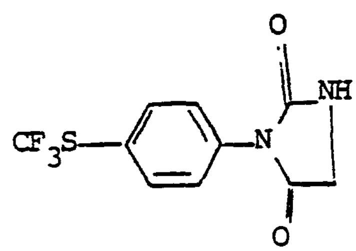

7,5 g (0,1 Mol) Aminoessigsäure werden unter Zusatz von 4 g (0,1 Mol) Ätznatron in 100 ml Wasser gelöst. Hierzu tropft man 21,9 g (0,1 Mol) 4-Trifluormethylmercaptophenylisocyanat, gelöst in 50 ml Aceton. Man rührt etwa 15 Min. und säuert mit Salzsäure an. Dabei fällt das Addukt aus, 30 g vom Schmelzpunkt 182-184°C.

Nach dem Trocknen wird die 4-Trifluormethylmercaptophenyl-carbamoyl-aminoessigsäure unter Zugabe von 2 ml konz. Schwefelsäure in 350 ml Xylol etwa 30 Min. lang am Wasserabscheider erhitzt. Nach Abdestillieren des Lösungsmittels erhält man nach Verrühren mit Wasser 21 g des 3-(4-Trifluormethylmercaptophenyl)-hydantoins vom Schmelzpunkt 117-120°C.

In gleicher Weise erhält man die Verbindungen der Formel (II)

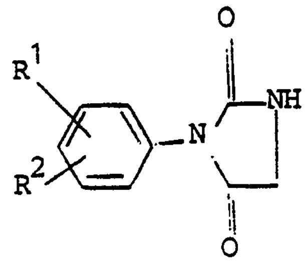

(II)

| Beispiel | $R^1$ | $R^2$ | Schmelzpunkt [°C] |
|---|---|---|---|
| b | 4-$CF_3$-O- | H | 140 |
| c | 4-$CF_2$Cl-O- | H | 137 |
| d | 4-$CF_3$-O- | 3-Cl | 120 |
| e | 4-$CF_2$Cl-S- | 3-Cl | 137 |

Beispiel f

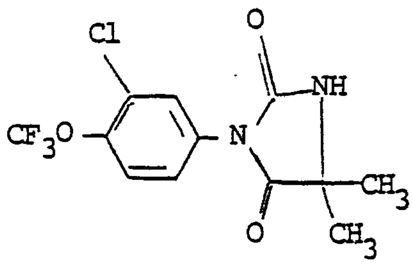

In eine Lösung von 17,1 g (0,072 Mol) 3-Chlor-4-trifluormethoxyphenylisocyanat in 100 ml Aceton tropft man 6,5 g (0,077 Mol) α-Aminoisobuttersäurenitril. Hierbei steigt die Temperatur bis etwa 45°C an. Nach Abklingen der Reaktion wird das Lösungsmittel im Vakuum abgedampft und der Rückstand anschließend mit 80 ml halbkonzentrierter Salzsäure 1 Stunde lang gekocht. Das hierbei entstehende 3-(3-Chlor-4-trifluormethoxyphenyl)-5,5-dimethyl-hydantoin wird aus Toluol unter Zusatz von Petrolether umkristallisiert. Man erhält 16 g mit dem Schmelzpunkt 104-107°C.

In ähnlicher Weise erhält man die Verbindungen

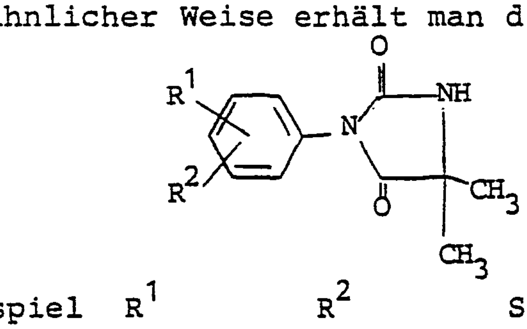

| Beispiel | $R^1$ | $R^2$ | Schmelzpunkt (°C) |
|---|---|---|---|
| g | 4-$CF_2Cl$-O- | 3-Cl | 134 |
| h | 4-$CF_3$-O- | H | 152 |
| i | 4-$CF_3$-S- | H | 140 |

Anwendungsbeispiele

In den nachfolgenden Beispielen werden die nachstehend aufgeführten bekannten Verbindungen als Vergleichssubstanzen eingesetzt:

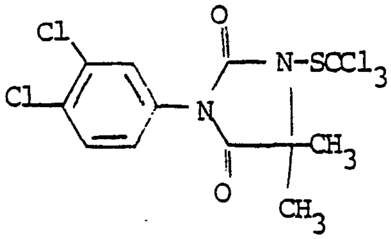

(A)

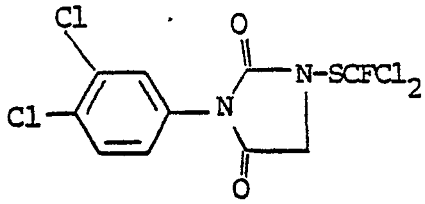

(B)

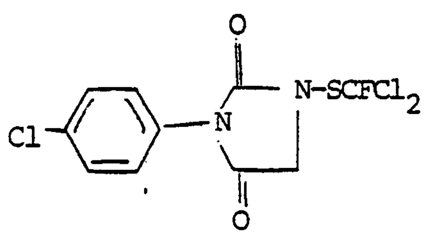

(C)

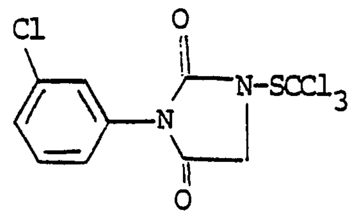

(D)

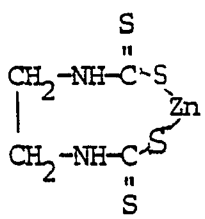

(E)

Beispiel A

Phytophthora-Test (Tomate) /protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 5, 3, 9, 1, 2, 4 und 7.

Beispiel B

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 7, 4, 2, 1, 10, 9, 3 und 5.

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 1 und 5.

Beispiel D

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäß folgender Herstellungsbeispiele: 3 und 9.

Patentansprüche

1. N-Sulfenylierte Hydantoine der Formel (I)

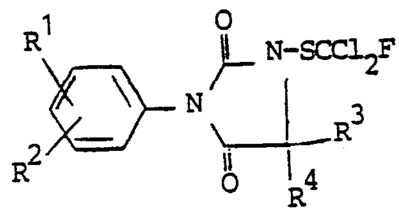

(I)

in welcher

$R^1$ für Trihalogenmethoxy oder Trihalogenmethylmercapto steht,

$R^2$ für Wasserstoff, Nitro oder Halogen steht und

$R^3$ und $R^4$ gleich oder verschieden sind und für Wasserstoff oder Alkyl stehen oder gemeinsam mit dem Ringkohlenstoff an dem sie stehen einen fünf- oder sechsgliedrigen Cycloalkylrest bilden.

2. N-Sulfenylierte Hydantoine gemäß Formel (I) in Anspruch 1, wobei

$R^1$ für Trichlormethoxy, Trifluormethoxy, Trichlormethylmercapto, Trifluormethylmercapto, Difluorchlormethoxy, Fluordichlormethoxy, Difluorchlormethylmercapto oder Fluordichlormethylmercapto und

$R^2$ für Wasserstoff, Nitro oder Chlor stehen, sowie

$R^3$ und $R^4$ gleich oder verschieden sind und für Wasserstoff oder Niederalkyl mit 1 bis 4 Kohlenstoffatomen stehen oder gemeinsam mit dem Ringkohlenstoff an dem sie stehen einen Cyclopentyl- oder Cyclohexylring bilden.

3. Verfahren zur Herstellung von N-sulfenylierten Hydantoinen der Formel (I)

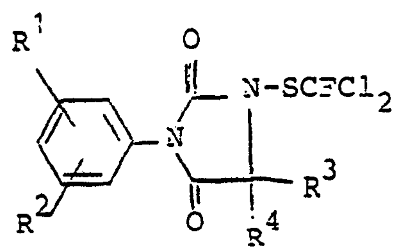

(I)

in welcher

$R^1$ für Trihalogenmethoxy oder Trihalogenmethylmercapto steht,

$R^2$ für Wasserstoff, Nitro oder Halogen steht und

$R^3$ und $R^4$ gleich oder verschieden sind und für Wasserstoff oder Alkyl stehen oder gemeinsam mit dem Ringkohlenstoff an dem sie stehen einen fünf- oder sechsgliedrigen Cycloalkylrest bilden, dadurch gekennzeichnet, daß man ein Hydantoin der Formel

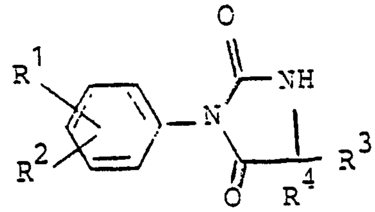

(II)

in welcher
$R^1$, $R^2$, $R^3$ und $R^4$ die oben angegebene Bedeutung haben, mit einem Dichlorfluormethansulfenylhalogenid der Formel

$$X\text{-}SCCl_2F \qquad (III)$$

in welcher
X für Halogen steht,

gegebenenfalls in Gegenwart eines säurebindenden Mittels und eines Verdünnungsmittels umsetzt.

4. Schädlingsbekämpfungsmittel, gekennzeichnet durch einen Gehalt an mindestens einem N-sulfenylierten Hydantoin der Formel (I) gemäß Ansprüchen 1 und 3.

5. Verwendung von N-sulfenylierten Hydantoinen der Formel (I) gemäß Ansprüchen 1 und 3 zur Bekämpfung von Schädlingen.

6. Verfahren zur Bekämpfung von Schädlingen, dadurch gekennzeichnet, daß man N-sulfenylierte Hydantoine der Formel (I) gemäß Ansprüchen 1 und 3 auf Schädlinge und/oder ihren Lebensraum einwirken läßt.

7. Verfahren zur Herstellung von Schädlingsbekämpfungsmitteln, dadurch gekennzeichnet, daß man N-sulfenylierte Hydantoine der Formel (I) gemäß Ansprüchen 1 und 3 mit Streckmitteln und/oder oberflächenaktiven Mitteln vermischt.

Europäisches Patentamt

# EUROPÄISCHER RECHERCHENBERICHT

0101800

Nummer der Anmeldung

EP 83 10 5502

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 3) |
|---|---|---|---|
| A | DE-A-2 658 270 (SUMITOMO) | | C 07 D 233/80<br>C 07 D 235/02<br>A 01 N 43/50<br>A 01 N 43/52 |
| | --- | | |
| A | DE-B-1 168 914 (BAYER) | | |
| | --- | | |
| A | DE-A-2 722 035 (BASF) | | |
| | ----- | | |
| | | | **RECHERCHIERTE SACHGEBIETE (Int. Cl 3)** |
| | | | C 07 D 233/00<br>C 07 D 235/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 29-11-1983 | DE BUYSER I.A.F. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82